# EUROPEAN PATENT APPLICATION

(11) **EP 4 706 542 A1**
(43) Date of publication of application: **11.03.2026**
(21) Application number: 24939215.0
(22) Date of filing: 24.05.2024
(51) Int. Cl.: A61B 5/389

(54) **SIGNAL ACQUISITION APPARATUS**

(71) Applicant: Ascend Technology Limited, Hong Kong 999077 (HK); Donghua University, Shanghai 200051 (CN)
(72) Inventor: ZHOU, Xin, Shenzhen, Guangdong 518108 (CN); WANG, Yongrong, Shanghai 200051 (CN); ZHANG, Jing, Shanghai 200051 (CN); LIAO, Fengyun, Shenzhen, Guangdong 518108 (CN); QI, Xin, Shenzhen, Guangdong 518108 (CN)
(74) Representative: Wang, Bo
(86) International application number: PCT/CN2024/095257
(87) International publication number: WO 2025/241186

(57) **Abstract**

Provided is a signal acquisition device. The signal acquisition device includes a wearable body, the wearable body includes: a plurality of electrodes in contact with a user's skin, the plurality of electrodes including a first electrode and a second electrode, the first electrode and the second electrode being configured to acquire an electromyography signal (EMG signal) of a target muscle of the user, and the first electrode and the second electrode being arranged at intervals along a muscle fiber direction of the target muscle; and, a base body, configured to carry the plurality of electrodes and position the plurality of electrodes at the target muscle; wherein, the base body includes a base yarn, the plurality of electrodes include conductive yarn, and the base body and the plurality of electrodes are formed by integrated weaving.

## Description

### TECHNICAL FIELD

The present disclosure relates to the field of signal acquisition, and in particular to a signal acquisition device.

### BACKGROUND

Monitoring of running exercise can be implemented using an electromyography (EMG) leg sleeve. The EMG leg sleeve is generally arranged along a muscle fiber direction to collect and monitor an electromyography signal (EMG signal) of a leg muscle. However, current EMG leg sleeves have poor wearing comfort. Due to the presence of an electrode structure, local elasticity is poor, and the wearing process is troublesome.

Therefore, it is desired to provide a signal acquisition device that ensures appropriate elasticity at various positions of a wearable body, offers low wearing difficulty and high wearing comfort, and is suitable for a wide range of users.

### SUMMARY

One or more embodiments of the present disclosure provide a signal acquisition device. The device includes a wearable body. The wearable body includes: a plurality of electrodes in contact with a user's skin and a base body. The plurality of electrodes include a first electrode and a second electrode, the first electrode and the second electrode being configured to acquire an electromyography signal (EMG signal) of a target muscle of the user, and the first electrode and the second electrode being arranged at intervals along a muscle fiber direction of the target muscle. The base body is configured to carry the plurality of electrodes and position the plurality of electrodes at the target muscle. The base body includes a base yarn, the plurality of electrodes include a conductive yarn, and the base body and the plurality of electrodes are formed by integrated weaving.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering indicates the same structure, wherein:
FIG. 1 is a schematic diagram illustrating a structure of a signal acquisition device according to some embodiments of the present disclosure.
FIG. 2 is a schematic diagram illustrating one or more waterproof layers according to some embodiments of the present disclosure.
FIG. 3 is a schematic diagram illustrating a partial view of a wearable body according to some embodiments of the present disclosure.
FIG. 4 is a schematic diagram illustrating different weaving manners according to some embodiments of the present disclosure.
FIG. 5 is a schematic diagram illustrating a partial view of a wearable body according to some embodiments of the present disclosure.
FIG. 6 is a schematic diagram illustrating a structure of a signal acquisition device according to other embodiments of the present disclosure.
FIG. 7 is a schematic diagram illustrating a structure of a fixing member according to some embodiments of the present disclosure.

### DETAILED DESCRIPTION

In order to provide a clearer understanding of the technical solutions of the embodiments described in the present disclosure, a brief introduction to the drawings required in the description of the embodiments is given below. It is evident that the drawings described below are merely some examples or embodiments of the present disclosure, and for those skilled in the art, the present disclosure may be applied to other similar situations without exercising creative labor. It should be understood that these exemplary embodiments are provided only to enable relevant field technicians to better understand and implement the present disclosure, and are not intended to limit the scope of the present disclosure in any way. Unless obviously obtained from the context or the context illustrates otherwise, the same numeral in the drawings refers to the same structure or operation.

As shown in the present disclosure and the claims, unless the context clearly indicates an exception, the terms "a", "an", "one", and/or "the" are not limited to the singular, but may also include the plural. In general, the terms "comprise", "comprises", and/or "comprising", "include", "includes", and/or "including", merely prompt to include steps and elements that have been clearly identified, and these steps and elements do not constitute an exclusive listing. The methods or devices may also include other steps or elements. The term "based on" means "based at least in part on." The term "one embodiment" means "at least one embodiment"; the term "another embodiment" means "at least one other embodiment".

In the description of the present disclosure, it should be understood that the orientation or positional relationship indicated by terms such as "front" and "rear" is based on the orientation or positional relationship shown in the drawings. This is only for convenience of describing the present disclosure and simplifying the description, and does not indicate or imply that the device or element must have a specific orientation or be constructed and operated in a specific orientation. Therefore, it should not be construed as a limitation on the present disclosure.

Furthermore, the terms "first" and "second" are used for descriptive purposes only, and should not be construed as indicating or implying relative importance or implicitly indicating the number of technical features. Thus, features defined with "first" and "second" may explicitly or implicitly include at least one such feature. In the description of the present disclosure, "a plurality of" means at least two, e.g., two, three, etc., unless otherwise explicitly and specifically defined.

In the present disclosure, unless otherwise explicitly specified and defined, terms such as "install", "connected", "connect", and "fix" should be understood broadly. For example, the connection may be a fixed connection, a detachable connection, or an integral connection. The connection may be a mechanical connection, an electrical connection, or a direct connection. The connection may be an indirect connection through an intermediate medium, an internal connection between two elements, or an interaction relationship between two elements, unless otherwise explicitly limited. For a person of ordinary skill in the art, the specific meanings of the above terms in the present disclosure can be understood according to specific situations.

Some embodiments of the present disclosure provide a signal acquisition device. In some embodiments, the signal acquisition device includes a wearable body. The wearable body includes a plurality of electrodes in contact with a user's skin. The plurality of electrodes includes a first electrode and a second electrode, the first electrode and the second electrode are configured to acquire an electromyography signal (EMG signal) of a target muscle of the user, and the first electrode and the second electrode are arranged at intervals along a muscle fiber direction of the target muscle. The first electrode and the second electrode may acquire electrical potentials on a skin surface at respective positions thereof. A potential difference between the acquired electrical potentials may be used to reflect the EMG signal of the target muscle. In some embodiments, the wearable body further includes a base body, the base body may carry the plurality of electrodes and position the plurality of electrodes at the target muscle. The base body includes a base yarn, the plurality of electrodes includes a conductive yarn, and the base yarn and the conductive yarn are woven together to achieve the integrated weaving of the base body and the plurality of electrodes. Through the integrated weaving of the base body and the plurality of electrodes, embodiments of the present disclosure enable the signal acquisition device to exhibit greater overall elasticity, making it easy to wear. Additionally, this approach significantly simplifies the preparation process of the base body and electrodes, achieves automation in production, and brings numerous advantages such as mass production, controllable quality, and excellent consistency. Furthermore, by selecting appropriate materials for the base yarn and conductive yarn, along with designing the weaving structure during the integrated weaving process, varying levels of elasticity can be achieved in different portions of the wearable body. This allows for precise control of pressure exerted on the user's skin across various portions of the wearable body.

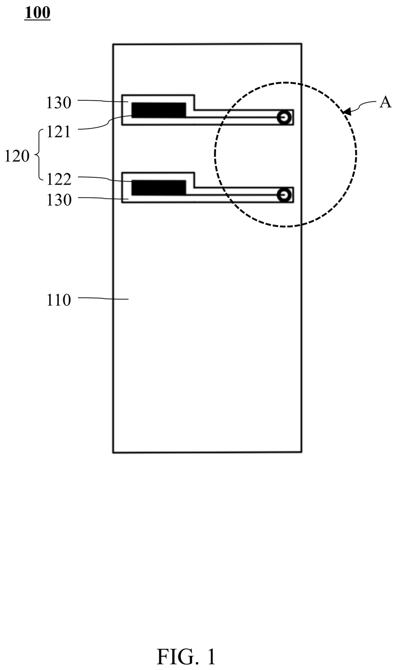

FIG. 1 is a schematic diagram illustrating a structure of a signal acquisition device according to some embodiments of the present disclosure. As shown in FIG. 1, a signal acquisition device 100 may include a wearable body.

The signal acquisition device 100 may acquire an electromyography signal (EMG signal) of a muscle of a user. For example, the signal acquisition device 100 may obtain the EMG signal of the muscle of the user through a plurality of electrodes in contact with the human skin. In some embodiments, the signal acquisition device 100 may include the wearable body (e.g., clothing), and the wearable body is configured to be worn on the body of the user. When the user wears the wearable body, the plurality of electrodes of the wearable body may be in contact with the user's skin to acquire the EMG signal on a skin surface. Exemplarily, a potential difference acquired by two electrodes arranged at intervals along a muscle fiber direction of a target muscle may characterize an EMG signal of the target muscle. In some embodiments, the wearable body may be in contact with a plurality of portions of the human body, e.g., a calf, a thigh, the hip, the waist, the back, the chest, a shoulder, the neck, etc., to acquire EMG signals of the plurality of portions. Merely by way of example, the wearable body is in contact with a leg of the user, and the signal acquisition device 100 is configured to acquire an EMG signal of a leg muscle of the user.

In some embodiments, the wearable body includes, but is not limited to, an upper garment (e.g., a T-shirt, a vest, a sleeveless shirt, a jacket, etc.), a shoulder protector, a waistband, a wrist protector, an elbow protector, a wristband, a knee protector, a skirt, pants (e.g., long pants, shorts, etc.), socks, etc. In some embodiments, the wearable body includes a strap. The strap may be fixed to a portion of the body of the user, the portion may be the waist, the chest, a wrist, an arm, an ankle, a leg, the neck, a finger, etc., by means of tying, sticking, etc. The present disclosure does not limit the form of the wearable body. Any device capable of using the present disclosure falls within the protection scope of the present disclosure. In some embodiments, the wearable body includes a base body 110 and a plurality of electrodes 120.

The base body 110 refers to a carrier for carrying components such as the plurality of electrodes 120, and the base body 110 positions the plurality of electrodes 120 at the target muscle of the human body. In some embodiments, the base body 110 may be implemented as a structure wearable on the leg, e.g., a leg strap (e.g., a full leg strap, a thigh strap, a calf strap, etc.), a leg sleeve (e.g., a full leg sleeve, a thigh sleeve, a calf sleeve, etc.), a sock (e.g., a calf sock, a thigh sock, pantyhose, etc.), pants, etc. In some embodiments, as shown in FIG. 1, the base body 110 forms the leg sleeve. The leg sleeve may be a closed ring structure with inherent elasticity, allowing it to elastically stretch along a radial direction thereof, and the leg sleeve may be entirely slipped onto the leg of the user, thus allowing for easy wear and high comfort. It should be noted that the base body 110 is not limited to the leg sleeve shown in FIG. 1, and may also include other forms (e.g., a sheet structure, a finger ring, etc.).

The plurality of electrodes 120 may acquire the EMG signal of the target muscle of the user. For example, the plurality of electrodes 120 may be fixed on the base body 110 and in contact with the user's skin, thereby acquiring the EMG signal on the skin surface. In some embodiments, the plurality of electrodes 120 may include a first electrode 121 and a second electrode 122. The first electrode 121 and the second electrode 122 may be configured to acquire the EMG signal of the target muscle of the user. The target muscle may be a single muscle, or a muscle group (e.g., a quadriceps muscle group including the rectus femoris, vastus lateralis, vastus medialis, and vastus intermedius). For example, the first electrode 121 and the second electrode 122 may be arranged at intervals along the muscle fiber direction of the target muscle and extend respectively along an extension direction perpendicular to the muscle fiber direction. Thus, the first electrode 121 and the second electrode 122 may acquire electrical potentials on the skin surface at respective positions thereof. A potential difference between the acquired electrical potentials may be used to reflect the EMG signal of the target muscle. In some embodiments, when the target muscle is the single muscle, since a plurality of other muscles exist around the target muscle, the EMG signal acquired by the plurality of electrodes 120 may include EMG signals of the target muscle and other muscles around it. Because the target muscle and other muscles around the target muscle are in a same muscle group, and EMG signals generated by muscles in the same muscle group are relatively similar, the EMG signal actually acquired by the plurality of electrodes 120 is close to the EMG signal of the target muscle, and the error is small. That is, the EMG signal actually acquired by the plurality of electrodes 120 may represent the EMG signal of the target muscle. It should be noted that, in other embodiments, a proportion of noise (i.e., EMG signals from other muscles around the target muscle) in the EMG signal acquired by the plurality of electrodes 120 may be reduced by means such as designing a shape of the plurality of electrodes 120 or performing algorithmic processing, so as to improve the accuracy of the signal acquisition device 100. The present disclosure does not impose excessive limitations on this.

In some embodiments, the plurality of electrodes 120 may acquire an EMG signal of a target muscle on a front side or a rear side of the leg of the user. Taking the calf as an example, the target muscle on the front side of the calf of the user may include a peroneus longus muscle, a tibialis posterior muscle, etc. The target muscle on the rear side of the calf may include a soleus muscle, a gastrocnemius muscle, etc. In some embodiments, muscles on the rear side of the leg (e.g., the gastrocnemius muscle) provide a main driving force for activities such as walking, running, and jumping of the body. By causing the plurality of electrodes 120 to acquire the EMG signal of the target muscle on the rear side of the leg of the user (e.g., an EMG signal of the gastrocnemius muscle), a running exercise of the user can be monitored relatively accurately. In some embodiments, the first electrode 121 and the second electrode 122 may be arranged at intervals along a muscle fiber direction of the gastrocnemius muscle and extend respectively along an extension direction perpendicular to the muscle fiber direction. That is, the first electrode 121 and the second electrode 122 may be disposed at the gastrocnemius muscle, and arranged at intervals along a height direction of the user and extend respectively along an extension direction perpendicular to the height direction of the user. The first electrode 121 and the second electrode 122 respectively acquire the electric potentials on the surface skin at respective positions thereof. A potential difference between the acquired electric potentials may be used to reflect the EMG signal of the gastrocnemius muscle.

In some embodiments, the plurality of electrodes 120 include a conductive yarn. The plurality of electrodes 120 acquire or transmit the electrical signal through the conductive yarn. The conductive yarn refers to a yarn having electrical conductivity. For example, the conductive yarn may be a conductive metal wire. In some embodiments, a conductive metal may be deposited on a surface of the yarn to form the conductive yarn. The yarn includes a textile material, for example, the textile material may include nylon, polyester, spandex, thermoplastic polyurethane elastomer (TPU), etc. Exemplarily, silver and/or silver chloride (with a chlorine content of 5%-15%) is deposited on the surface of the yarn to form the conductive yarn. In some embodiments, the plurality of electrodes 120 may be woven from the conductive yarn.

In some embodiments, the base body 110 may include a base yarn. Because the base body 110 carries the plurality of electrodes 120, in order to avoid an interference from the base body 110 on the signal acquisition of the plurality of electrodes 120 (e.g., the first electrode 121 and the second electrode 122) and affect accuracy of the acquisition of the EMG signal, in some embodiments, the base yarn may include an insulating yarn. An arrangement of the insulating yarn prevents the base body 110 from receiving an EMG signal of the human skin, and avoids an interference from the base body 110 on an operation of the plurality of electrodes 120 (e.g., the first electrode 121 and the second electrode 122). In order to provide the wearable body with elasticity, so that the wearable body in a worn state may fit the user's skin relatively well to facilitate the acquisition of the EMG signal by the plurality of electrodes 120, the base yarn may include an elastic yarn. The elastic yarn may provide the base body 110 with elasticity. In some embodiments, the base yarn may have both elasticity and insulating properties at the same time, i.e., the base yarn may include an elastic insulating yarn. In some embodiments, the base body 110 and the plurality of electrodes 120 are formed by integrated weaving. That is, the conductive yarn and the base yarn are formed by integrated weaving. For example, an intarsia four-feed machine is configured to achieve the integrated weaving of the conductive yarn and the base yarn. For example, a designed weaving structure drawing is imported into the four-feed machine to perform automatic weaving of the conductive yarn and the base yarn. The integrated weaving may be understood as a process of continuously weaving using a plurality of types of yarns to obtain an integral fabric. Exemplarily, when the fabric includes regions that need to be woven separately by different types of yarns, the integrated weaving manner may employ a transition weaving technique (e.g., tuck weaving) to continuously weave the regions without requiring individual pre-fabrication of each region in advance and then "splice" the regions together through a post-processing manner (e.g., sewing or bonding).

By integrally weaving the base body 110 and the plurality of electrodes 120, the embodiments of the present disclosure ensure that the entire structure possesses elasticity and consistency, enabling it to adapt to the user's body and thereby enhancing the wearing experience. The elasticity of the base body 110 enables the plurality of electrodes 120 to fit tightly against the user's skin, ensuring accuracy of the signal acquisition. Using the integrated weaving technique can simplify the manufacturing process, realize production automation of the signal acquisition device 100, and offer advantages such as ease of mass production, controllable quality, and excellent consistency.

An average blood pressure of capillaries on a surface of human skin is approximately 7.85 kPa. When the pressure exerted by the clothing on the human body in the worn state is too high (e.g., close to or greater than the average blood pressure), it may obstruct blood flow, causing difficulty in blood flow or even stopping blood flow. When the pressure exerted by the clothing on the human body in the worn state is too low, it may cause the clothing to be worn loosely, easily slip off, and affect wearing comfort and stability. In some embodiments, in order to provide the signal acquisition device 100 with relatively high wearing comfort and stability, in the worn state, a pressure exerted by the signal acquisition device 100 on the human body may range from 1.96 kPa to 3.92 kPa. Through the integrated weaving technique, thicknesses and/or yarn densities of different regions of the wearable body of the signal acquisition device 100 can be adjusted, thereby adjusting elastic forces at various locations of the wearable body, so that the wearable body in the worn state can fit the user's skin relatively well, and at the same time, the pressure exerted by the wearable body on the user's human body in the worn state is appropriate. Specifically, the greater a thickness of a certain region on the wearable body, the greater an elastic force of the region and the stronger the elasticity. The smaller a thickness of a certain region on the wearable body, the smaller an elastic force of the region and the weaker the elasticity. The greater a density of the elastic yarn in a certain region on the wearable body, the greater an elastic force of the region and the stronger the elasticity. The smaller a density of the elastic yarn in a certain region on the wearable body, the smaller an elastic force of the region and the weaker the elasticity. At the same time, through the design of the elastic force of the wearable body, the wearable body is provided with relatively good extensibility, so that the signal acquisition device 100 can be adapted to different groups of people, improving the applicable scope of the signal acquisition device 100.

During the operation of the signal acquisition device 100, due to external environmental factors (e.g., rain, etc.) and user personal factors (e.g., sweating during exercise, etc.), the wearable body may become wet by liquid. In order to prevent different electrodes 120 (e.g., the first electrode 121 and the second electrode 122) from being electrically connected to each other due to a wet wearable body, in some embodiments, as shown in FIG. 1, the wearable body further includes one or more waterproof layers 130. The one or more waterproof layers 130 may isolate an abnormal conduction between the plurality of electrodes 120 due to wetness, thereby avoiding the wet wearable body from affecting accuracy of the acquisition of the EMG signal. In some embodiments, the one or more waterproof layers 130 include a waterproof yarn. The waterproof yarn may respectively surround different electrodes 120. For example, the waterproof yarn may surround the first electrode 121 and/or the second electrode 122, so as to isolate the first electrode 121 from the second electrode 122. In some embodiments, the one or more waterproof layers 130, the base body 110 and the plurality of electrodes 120 are formed by integrated weaving. That is, the waterproof yarn, the conductive yarn, and the base yarn are formed by integrated weaving. The integrated weaving can improve flatness of the surface of the wearable body, and can simplify the manufacturing process, realize production automation of the signal acquisition device 100, and offer advantages such as ease of mass production, controllable quality, and excellent consistency.

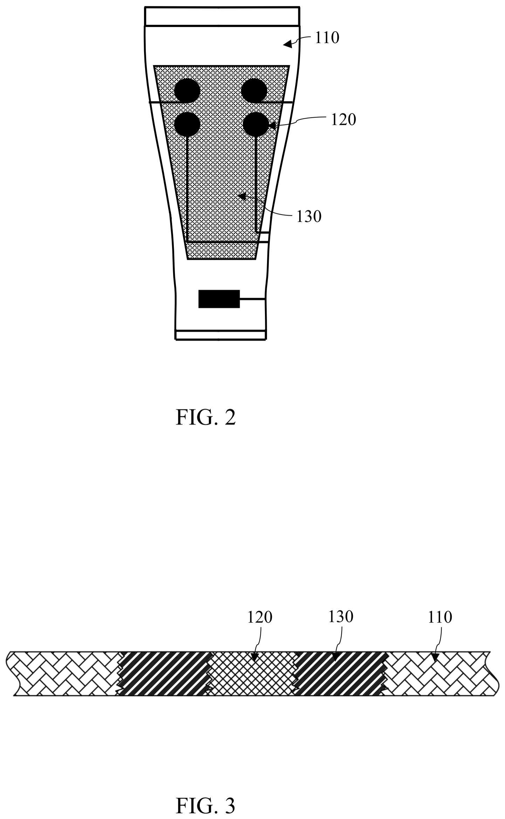

More descriptions regarding data and specifications of yarns such as the waterproof yarn and the conductive yarn may be found in subsequent content of the present disclosure (e.g., content related to FIG. 2).

It should be noted that the above description regarding FIG. 1 is provided merely for illustrative purposes and is not intended to limit the scope of the present disclosure. For those of ordinary skill in the art, various changes and modifications can be made according to the guidance of the present disclosure. For example, in some embodiments, the signal acquisition device 100 may further include one or more components (e.g., a circuit board, a battery, Bluetooth, an amplifier, a memory, an inertial sensor, etc.). Optionally or additionally, when the user wears the signal acquisition device 100, the one or more components may be located at an outer side of the leg, the waist, etc. These changes and modifications do not depart from the scope of the present disclosure.

In some embodiments, by designing different parameters of the conductive yarn, the conductive yarn may be provided with corresponding usage performance. Exemplarily, by designing a thickness of the conductive yarn, the conductive yarn may be adapted to different processing machines. By designing a strength of the conductive yarn, the conductive yarn may be prevented from breaking easily when entering a processing machine for integrated weaving. By designing an elastic modulus of the conductive yarn, the conductive yarn can be suitable for processing requirements of different processing machines, and it is also convenient to subsequently control elasticity of different regions of the wearable body of the signal acquisition device 100. By designing conductivity of the conductive yarn, the plurality of electrodes 120 can have good conductivity, and sensitivity of the plurality of electrodes 120 can be improved. By designing tolerance of the conductive yarn, performance and service life of the signal acquisition device 100 can be improved, and a probability of the signal acquisition device 100 having problems such as loss of conductivity, deformation, or discoloration can be reduced.

In some embodiments, the conductive yarn may include, but is not limited to, metal conductive yarn, non-metal conductive yarn, etc. Exemplarily, the metal conductive yarn may include a silver-plated nylon low-elastic yarn, a silver-plated nylon yarn, a silver fiber spandex covered yarn, a stainless steel yarn, or the like. The non-metal conductive yarn may include a graphene polyester conductive filament, a graphene polyester staple fiber yarn, or the like. The silver-plated nylon low-elastic yarn is made by using a pre-oriented yarn as a raw yarn for stretching and false-twist texturing. The silver-plated nylon yarn is made by plating a silver ion layer on a surface of a nylon filament. The silver fiber spandex covered yarn is an elastic yarn formed by covering an elongated spandex filament in a spiral manner with the silver-plated nylon low-elastic yarn. The stainless steel yarn is made by single-filament drawing or bundle drawing to make it fibrous. The graphene polyester conductive filament is made by using a polyester filament as a base and dip-coating a surface with a graphene slurry to make it conductive. The graphene polyester staple fiber yarn is made by adding graphene to a polyester masterbatch.

In some embodiments, from a perspective of yarn uniformity, the silver-plated nylon low-elastic yarn, the silver-plated nylon yarn, the silver fiber spandex covered yarn, the stainless steel yarn, the graphene polyester conductive filament, and the graphene polyester staple fiber yarn all have good yarn uniformity. The difficulty of integrated weaving is relatively low, a yarn density of a finished product (e.g., the wearable body) after integrated weaving is relatively uniform everywhere, and the finished product is not prone to force tearing and is convenient to control elasticity of different regions. From a perspective of tensile properties, the silver-plated nylon yarn, the silver fiber spandex covered yarn, the graphene polyester conductive filament, and the graphene polyester staple fiber yarn all have good tensile properties, which is beneficial for weaving elastic and flexible fabrics. The stainless steel yarn has poor tensile properties and high rigidity, is not easy to bend into loops, and is prone to cause weaving needle breakage during machine weaving. From a perspective of conductivity, the metal conductive yarns (the stainless steel yarn, the silver-plated nylon low-elastic yarn, the silver-plated nylon yarn, and the silver fiber spandex covered yarn) all have good conductivity.

In summary, in some embodiments, to facilitate machine processing and weaving, and to enable the processed electrodes 120 to have good performance, the conductive yarn may include at least one of the silver-plated nylon low-elastic yarn, the silver-plated nylon yarn, and the silver fiber spandex covered yarn.

In some embodiments, the conductive yarn is at least made by winding a conductive fiber (e.g., a carbon fiber, a metal fiber, etc.) around an elastic fiber (e.g., polyurethane, nylon, etc.), so that the conductive yarn has both conductivity and elasticity. Under a premise of being able to acquire the EMG signal, user wearing experience is improved. In other embodiments, the conductive yarn may also be at least made by coating a periphery of the conductive fiber with an elastic film (e.g., a polyurethane film). When the elastic film or the elastic fiber is in a natural state, the conductive fiber is in a relaxed state. That is, the conductive fiber may have some curved structures. In the worn state, the wearable body is deformed, the elastic film or the elastic fiber is stretched, and the curved structures of the conductive fiber are reduced. When the conductive fiber has no curved structure, the elastic conductive yarn is stretched to a maximum deformation.

By using the elastic conductive yarn, the embodiments of the present disclosure improve the elasticity of the plurality of electrodes 120, improve the wearing comfort of the signal acquisition device 100, and improve a signal-to-noise ratio of the EMG signal acquired by the signal acquisition device 100 and the stability of the signal acquisition.

In some embodiments, the conductive yarn may be subjected to waterproof and insulating treatment so that the conductive yarn has waterproof and insulating properties, thereby avoiding conduction between the plurality of electrodes 120 and affecting the accuracy of the acquisition of the EMG signal. In some embodiments, an insulating substance (e.g., EVA) may be wrapped around the conductive yarn. A portion that needs to be conductive (a portion where the conductive yarn contacts the user's skin) is processed. For example, a cladding of the portion is dissolved or removed to achieve a conductive effect, and the remaining cladding forms the one or more waterproof layers 130. After the insulating substance is coated on the surface of the conductive yarn, the elasticity of the conductive yarn disappears, making it difficult for a surface of the plurality of electrodes 120 to closely fit the skin. Furthermore, an excessively high temperature during the process of coating the insulating substance may cause a breakage of a spandex filament in the conductive yarn, affecting the elasticity of the conductive yarn.

FIG. 2 is a schematic diagram illustrating one or more waterproof layers according to some embodiments of the present disclosure.

In some embodiments, as shown in FIG. 2, the waterproof yarn, the conductive yarn, and the base yarn are formed by integrated weaving. The base yarn of the base body 110 surrounds the conductive yarn. The conductive yarn is woven to form the plurality of electrodes 120. The waterproof yarn is woven to form the one or more waterproof layers 130. The one or more waterproof layers 130 are arranged to surround the plurality of electrodes 120, thereby isolating each of the plurality of electrodes 120 from the base body 110, avoiding abnormal conduction between the plurality of electrodes 120, and affecting accuracy of the acquisition of the EMG signal. In some embodiments, the one or more waterproof layers 130 may be arranged to completely surround the plurality of electrodes 120. The waterproof yarn is used for local weaving to divide waterproof regions to achieve a purpose of preventing conduction between the plurality of electrodes 120, thereby solving a waterproof problem at a yarn level. In some embodiments, if a waterproof film is used for waterproof treatment of the plurality of electrodes 120, since the base body 110 has elasticity and extensibility as a woven fabric, and elasticity and extensibility of the waterproof film are difficult to be consistent with those of the base body 110 as the woven fabric, when the base body 110 is deformed and extended, deformation degrees of the base body 110 and the waterproof film may be different, which may cause the waterproof film to detach from the base body 110 or be damaged, thereby affecting a waterproof effect. By using the waterproof yarn for local weaving to divide the waterproof regions and solving the waterproof problem at the yarn level, a problem of poor waterproof effect caused by a mismatch in mechanical properties between the woven fabric and the waterproof film can be better avoided.

In some embodiments, a production process of the waterproof yarn may include two techniques: (1) adding a waterproof masterbatch to a raw liquid during a spinning process to achieve a waterproof and anti-siphon effect of the yarn; (2) impregnating the yarn in a waterproof finishing agent and then performing high-temperature drying. The waterproof yarn produced by technique (1) has a good waterproof effect, a high wash durability, and good abrasion resistance. The waterproof yarn produced by technique (2) has a good waterproof effect and can achieve an excellent anti-siphon effect. In some embodiments, a production technique of the waterproof yarn may include: making the yarn from the waterproof masterbatch and coating the surface of the yarn with spandex, thereby imparting elasticity to the yarn while maintaining optimal waterproof performance, facilitating integrated weaving and an elastic design of the wearable body.

FIG. 3 is a schematic diagram illustrating a partial view of a wearable body according to some embodiments of the present disclosure. As shown in FIG. 3, the one or more waterproof layers 130 and the base body 110 are formed by integrated weaving in a coplanar arrangement.

In some embodiments, the base body 110 is formed by weaving the base yarn. The one or more waterproof layers 130 are formed by weaving the waterproof yarn, etc.. The one or more waterproof layers 130 and the base body 110 are formed by weaving in the coplanar arrangement. An outer peripheral side of the one or more waterproof layers 130 is connected to the base body 110, so that an overall feel of the wearable body is flat and dense, thereby facilitating close contact between the wearable body and the human skin, and facilitating the acquisition of the EMG signal by the plurality of electrodes 120.

A connection between the outer peripheral side of the one or more waterproof layers 130 and the base body 110 may be achieved by one or more weaving manners. In some embodiments, the connection between the one or more waterproof layers 130 and the base body 110 may be achieved by one or more of weaving manners such as tucking, double looping, and staggered connection.

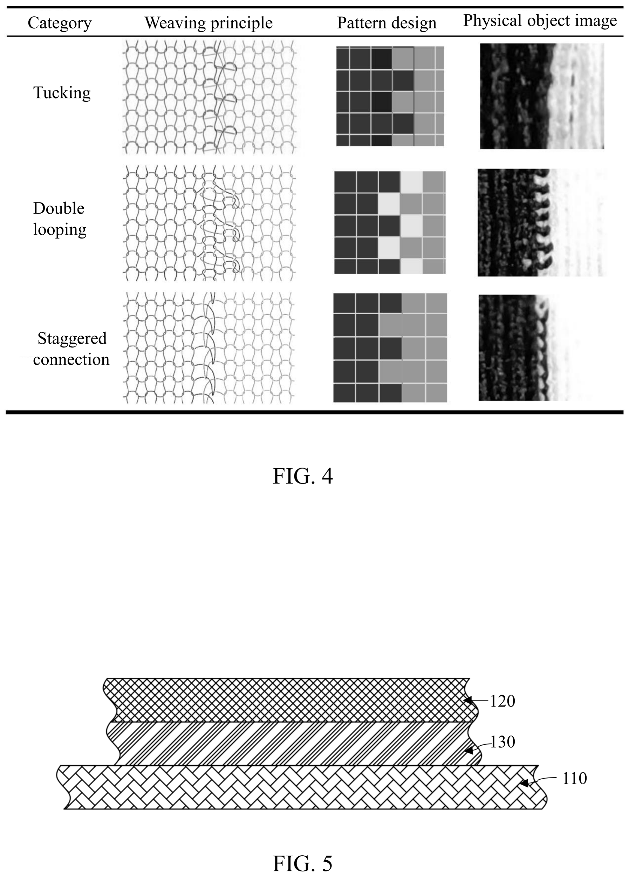

FIG. 4 is a schematic diagram illustrating different weaving manners according to some embodiments of the present disclosure. FIG. 4 shows weaving principles, design patterns, and woven objects of three weaving manners: tucking, double looping, and staggered connection.

As shown in FIG. 4, when the tucking manner is used for splicing, a transition region between different regions uses a high needle position on a left side and a half needle position on a right side, respectively. On the half needle position, an old loop on a weaving needle is not cast off while new yarn is hooked and sunk to form a new loop, and then the old loop and the new loop are cast off together to complete the tucking connection. The tucking manner increases a thickness of a junction region, resulting in an overall flat and dense hand feel and good firmness and abrasion resistance. When the double looping manner is used for splicing, both left and right sides use the high needle position, with two consecutive casting-off actions at a junction to complete the connection. Splicing by the double looping manner makes the junction region longer, forms obvious pores, and forms raised edges with a wrinkled texture. The staggered connection manner uses a floating thread to reduce a size of an eyelet, resulting in the junction is flat with a uniform thickness. In some embodiments, the one or more waterproof layers 130 and the base body 110 are formed by weaving in the tucking manner, which can make the junction between the one or more waterproof layers 130 and the base body 110 flat and dense, offering the good firmness and abrasion resistance, and can effectively prevent the connection from breaking during use.

In some embodiments, to avoid the abnormal conduction between the plurality of electrodes 120 (e.g., between the first electrode 121 and the second electrode 122), the one or more waterproof layers 130 may be provided to isolate the different electrodes 120. The plurality of electrodes 120 are provided on the base body 110. Correspondingly, the one or more waterproof layers 130 include a plurality of waterproof layers. The plurality of waterproof layers 130 and the plurality of electrodes 120 are provided in a one-to-one correspondence. Each of the one or more waterproof layers 130 isolates the corresponding electrode 120. In some embodiments, each of one or more waterproof layers 130 is arranged to surround a corresponding electrode 120. Each of the plurality of electrodes 120 is connected to an inner peripheral side of a corresponding waterproof layer 130. In some embodiments, as shown in FIG. 3, each of the plurality of electrodes 120 and a corresponding waterproof layer 130 are formed by weaving in the coplanar arrangement.

In some embodiments, as shown in FIG. 3, the base body 110, the one or more waterproof layers 130, and the plurality of electrodes 120 may be sequentially formed by weaving in the coplanar arrangement. The one or more waterproof layers 130 may separate the base body 110 and the plurality of electrodes 120, thereby preventing the base body 110 from wetting the plurality of electrodes 120 and causing the abnormal conduction between the different electrodes 120. On the other hand, under a condition that an overall thickness of the wearable body is constant, by weaving the base body 110, the one or more waterproof layers 130, and the plurality of electrodes 120 in the coplanar arrangement, the thickness of the plurality of electrodes 120 can be effectively increased, while a contact pressure between the plurality of electrodes 120 and the user's skin can be better reduced, thereby improving the wearing comfort of the signal acquisition device 100.

In some embodiments, a connection between each of the plurality of electrodes 120 and an inner peripheral side of a corresponding waterproof layer 130 may be achieved by one or more weaving manners. In some embodiments, a connection between the each of the plurality of electrodes 120 and the corresponding waterproof layer 130 may be achieved by one or more of weaving manners such as tucking, double looping, and staggered connection. In some embodiments, as shown in FIG. 4, each of the plurality of electrodes 120 is woven on the corresponding waterproof layer 130 in the tuck manner, which makes the junction between the each of the plurality of electrodes 120 and the corresponding waterproof layer 130 flat, dense, firm, and abrasion-resistant, thereby effectively preventing the connections from being damaged or broken during use.

In some embodiments, in the coplanar arrangement, in a direction toward the user's skin, the each of the plurality of electrodes 120 protrudes beyond the corresponding waterproof layer 130. That is, in the direction toward the user's skin, a thickness dimension of each of the plurality of electrodes 120 is greater than a thickness dimension of the corresponding waterproof layer 130, so that the each of the plurality of electrodes 120 protrudes beyond the corresponding waterproof layer 130. Thus, when the base body 110 and the one or more waterproof layers 130 contact the skin, the plurality of electrodes 120 contacts the skin more closely, and the signal acquisition performance is enhanced.

In some embodiments, to enable the one or more waterproof layers 130 to isolate the plurality of electrodes 120 from the base body 110, the each of the plurality of electrodes 120 is woven on a side of the corresponding waterproof layer 130 facing the user's skin. That is, the each of the plurality of electrodes 120 and the corresponding waterproof layer 130 are arranged in a stacked manner along the direction toward the user's skin. In some embodiments, in a direction perpendicular to the direction toward the user's skin, an area of the corresponding waterproof layer 130 is greater than an area of each of the plurality of electrodes 120, thereby ensuring a waterproof isolation effect of the one or more waterproof layers 130.

By weaving connecting the each of the plurality of electrodes 120 to the side of the corresponding waterproof layer 130 facing the user's skin in the stacked manner, the each of the plurality of electrodes 120 is arranged to protrude beyond the corresponding waterproof layer 130. When the base body 110 and the one or more waterproof layers 130 are in contact with the skin, the plurality of electrodes 120 contact the skin more closely, and the signal acquisition is enhanced.

FIG. 5 is a schematic diagram illustrating a partial view of a wearable body according to some embodiments of the present disclosure. As shown in FIG. 5, in some embodiments, the one or more waterproof layers 130 are woven connected to a side (i.e., an inner surface) of the base body 110 facing the user's skin. That is, the one or more waterproof layers 130 and the base body 110 are arranged in the stacked manner along the direction toward the user's skin, so that the one or more waterproof layers 130 protrude beyond the base body 110 to increase a vertical spacing between the plurality of electrodes 120 and the base body 110, which can better avoid the abnormal conduction between the different electrodes 120 caused by the base body 110 in a wet state.

In some embodiments, each of the plurality of electrodes 120 are woven on a side of the corresponding waterproof layer 130 facing away from the base body 110. That is, each of the plurality of electrodes 120 and the corresponding waterproof layer 130 are arranged in the stacked manner along the direction toward the user's skin, so that each of the plurality of electrodes 120 protrudes beyond the corresponding waterproof layer 130. Thus, when the base body 110 contacts the skin, the plurality of electrodes 120 contact the skin more closely, and the signal acquisition is enhanced. In some embodiments, in the direction perpendicular to the direction toward the user's skin, the area of the corresponding waterproof layer 130 is greater than or equal to the area of each of the plurality of electrodes 120, to ensure the waterproof isolation effect of the one or more waterproof layers 130.

In other embodiments, when the base body 110 and the one or more waterproof layers 130 are arranged in the stacked manner along the direction toward the user's skin, each of the plurality of electrodes 120 and the corresponding waterproof layer 130 are formed by weaving in the coplanar arrangement. The present disclosure does not impose excessive limitations on this.

In some embodiments, when the plurality of electrodes 120, the one or more waterproof layers 130, and the base body 110 are sequentially formed by weaving in the coplanar arrangement, concave-convex shapes are woven on surfaces of the one or more waterproof layers 130 close to and/or away from the skin, to increase the elasticity of the one or more waterproof layers 130. During wearing, the one or more waterproof layers 130 are stretched, and a degree of concavity and convexity on the surface of the one or more waterproof layers 130 is reduced. Meanwhile, the waterproof effect is not weakened, which is beneficial to ensuring a liquid barrier effect of the one or more waterproof layers 130. In some embodiments, when the one or more waterproof layers 130 and the base body 110 are formed by weaving in the coplanar arrangement, and each of the plurality of electrodes 120 and the corresponding waterproof layer 130 are formed by weaving in the stacked manner, the surfaces of the one or more waterproof layers 130 away from the skin are woven to form the concave-convex shape, which facilitates the connection of the plurality of electrodes 120 to the surfaces of the one or more waterproof layers 130 close to the skin, while increasing the elasticity of the one or more waterproof layers 130. In some embodiments, when each of the plurality of electrodes 120 and the corresponding waterproof layer 130 are formed by weaving in the coplanar arrangement or the stacked manner, a surface of the plurality of electrodes 120 close to the skin is woven to form the concave-convex shape, which increases an effective area of a region of the plurality of electrodes 120. When the plurality of electrodes 120 is stretched, a contact area between the plurality of electrodes 120 and the skin is increased, friction with the skin is increased, and a signal-to-noise ratio is increased. In some embodiments, when the plurality of electrodes 120, the one or more waterproof layers 130, and the base body 110 are sequentially formed by weaving in the coplanar arrangement, two surfaces of the plurality of electrodes 120 close to and away from the skin are simultaneously woven to form the concave-convex shape, which further increases the elasticity of the plurality of electrodes 120. When the plurality of electrodes 120 are stretched, the contact area with the skin is increased.

In some embodiments, the wearable body does not include the one or more waterproof layers. Each of the plurality of electrodes 120 includes a first region and a second region, the first region is connected to the base body 110 through the second region, the first region contacts the user's skin to acquire an electrical signal, and the second region is electrically insulated from the user's skin. The first region here is similar to the aforementioned electrode 120 and has conductivity. The second region is similar to the aforementioned one or more waterproof layers 130 and has waterproof insulation. The second region of the each of the plurality of electrodes 120 may isolate the first region of the electrode 120 from first regions of other electrodes 120. In some embodiments, the second region of the each of the plurality of electrodes 120 surrounds the first region of the electrode 120. That is, the second region and the first region of the each of the plurality of electrodes 120 are formed by weaving in the coplanar arrangement. In some embodiments, the second region of the each of the plurality of electrodes 120 and the base body 110 are formed by weaving spliced in the coplanar arrangement or the stacked manner.

In embodiments of the present disclosure, by eliminating the need for a separate waterproof layer 130 and implementing regionally distributed electrodes 120, the second region achieves waterproof isolation between the first region and the base body 110. This integrated approach eliminates the requirement for the waterproof yarn during the weaving process of the wearable body, thereby reducing material costs and simplifying manufacturing complexity.

In some embodiments, in the direction toward the user's skin, for each of the plurality of electrodes 120, the first region protrudes beyond the second region. That is, in the direction toward the user's skin, for each of the plurality of electrodes 120, a thickness dimension of the first region is greater than a thickness dimension of the second region, so that the first region protrudes beyond the second region. When the base body 110 contacts the skin, under an elastic effect of the base body 110, the first region contacts the skin more closely, and the signal acquisition is enhanced. At the same time, the first region protrudes beyond the second region, which indirectly increases a contact area between the first region and the skin, facilitating the signal acquisition.

In some embodiments, the first region and the second region included in the each of the plurality of electrodes 120 both include the conductive yarn. The conductive yarn is subjected to waterproof insulation treatment, so that the conductive yarn itself has waterproof insulation. For example, after the conductive yarn is coated with a waterproof insulating film (e.g., EVA), the plurality of electrodes 120 are formed by weaving, a portion requiring conductivity (i.e., the first region) is processed. For example, by dissolving or removing the waterproof insulating film of the conductive yarn in the first region, the conductive yarn in the first region may directly contact the skin to acquire the EMG signal. The conductive yarn in the second region retaining the waterproof insulating film has a waterproof insulation effect. As another example, after the conductive yarn is woven to form the plurality of electrodes 120, the second region is directly subjected to the waterproof treatment (e.g., immersion, application of a waterproof solvent, etc.), and the first region is not subjected to the waterproof treatment.

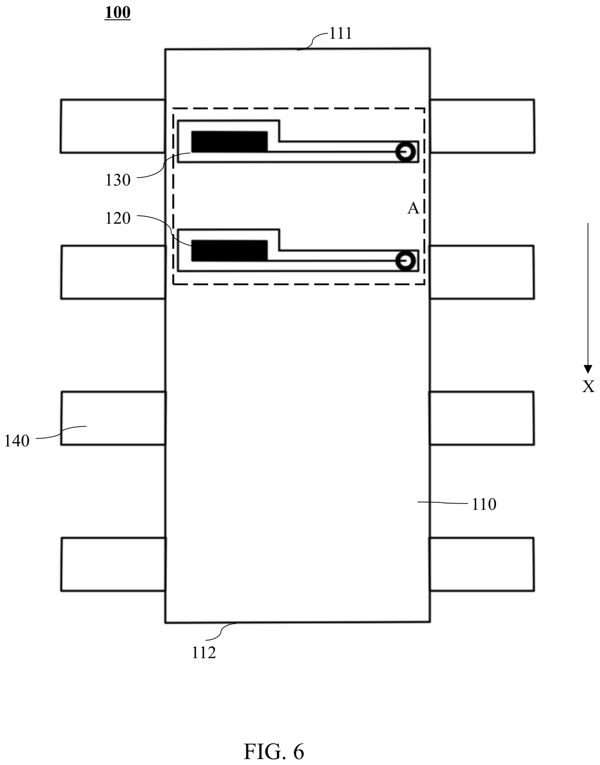

FIG. 6 is a schematic diagram illustrating a structure of a signal acquisition device according to other embodiments of the present disclosure.

In some embodiments, as shown in FIG. 6, the wearable body includes a plurality of straps 140 connected to the base body 110. The plurality of straps 140 are arranged at intervals along the muscle fiber direction (e.g., an X direction in FIG. 6). Each of the plurality of strap 140 extends in a direction perpendicular to the muscle fiber direction. In the worn state, the base body 110 extends along the muscle fiber direction. The plurality of straps 140 and the plurality of electrodes 120 are arranged at intervals along the muscle fiber direction. Merely by way of example, when the signal acquisition device 100 is worn on a leg, the base body 110 wraps a portion of the leg. Each of the plurality of straps 140 is fixedly connected to two sides of the base body 110 perpendicular to the muscle fiber direction, so that the base body 110 is fixed to the leg. In some embodiments, a size of each of the plurality of straps 140 is individually adjustable, so that the wearable body better adapts to the body of the user, thereby ensuring the base body 110 to closely fit the user's skin to ensure that the plurality of electrodes 120 can acquire the EMG signal.

In some embodiments, different portions of the base body 110 have different elasticity, so that various regions of the wearable body respectively fit corresponding regions of the body of the user, to ensure the signal acquisition effect. In some embodiments, a portion of the base body 110 close to or surrounding the plurality of electrodes 120 has greater elasticity, thereby enabling the plurality of electrodes 120 to fit the user's skin, ensuring the accuracy of the signal acquisition, it can also compensate for reduced elasticity caused by the plurality of electrodes 120 and the one or more waterproof layers 130, thereby ensuring that the base body 110 can fit lines of different positions on the body of the user, and ensuring a robust signal acquisition. In some embodiments, the base body 110 includes an overlapping region (e.g., a dotted region A in FIG. 6) overlapping with the plurality of electrodes 120 in a direction perpendicular to the muscle fiber direction (i.e., a direction perpendicular to the X direction in FIG. 6). The base body 110 includes a non-overlapping region (e.g., a region of the base body 110 other than the dotted region A in FIG. 6) staggering the plurality of electrodes 120 in the direction perpendicular to the muscle fiber direction (i.e., the direction perpendicular to the X direction in FIG. 6). As shown in FIG. 6, the overlapping region and the non-overlapping region both extend along the direction perpendicular to the muscle fiber direction (i.e., the direction perpendicular to the X direction in FIG. 6). The overlapping region and the non-overlapping region are adjacently distributed along the muscle fiber direction (i.e., the X direction in FIG. 6). Exemplarily, when the base body 110 forms a leg sleeve, in a wearing direction of the leg sleeve (e.g., the muscle fiber direction of the target muscle, the X direction in FIG. 6), the base body 110 includes an annular overlapping region located in the same dimension as the plurality of electrodes 120, a region of the base body 110 staggering the plurality of electrodes 120 in the X direction is the non-overlapping region. In some embodiments, in the direction perpendicular to the muscle fiber direction, a portion of the base body 110 in the same dimension as the plurality of electrodes 120 and the one or more waterproof layers 130 has greater elasticity, which can compensate for the reduced elasticity caused by the plurality of electrodes 120 and the one or more waterproof layers 130, so that the wearable body can better fit the worn portion.

In the human circulatory system, blood return occurs more readily in regions closer to the heart and encounters greater resistance farther from the heart. To reduce an impact of the wearable body of the signal acquisition device 100 on blood return in the worn state, elasticity of a distal end 112 of the base body 110 away from the user's heart is greater than elasticity of a proximal end 111 of the base body 110 close to the user's heart. Through the arrangement of greater elasticity at the distal end 112 of the base body 110, a squeezing force of the distal end 112 of the base body 110 on the human body is reduced, thereby facilitating blood return to the heart and improving the wearing comfort and safety of the signal acquisition device 100.

In some embodiments, to make the elasticity of the distal end 112 greater than the elasticity of the proximal end 111, during the integrated weaving process, a thickness of the distal end 112 is made greater than a thickness of the proximal end 111. In other embodiments, to make the elasticity of the distal end 112 greater than the elasticity of the proximal end 111, during the integrated weaving process, a density of the base yarn at the distal end 112 is greater than a density of the base yarn at the proximal end 111.

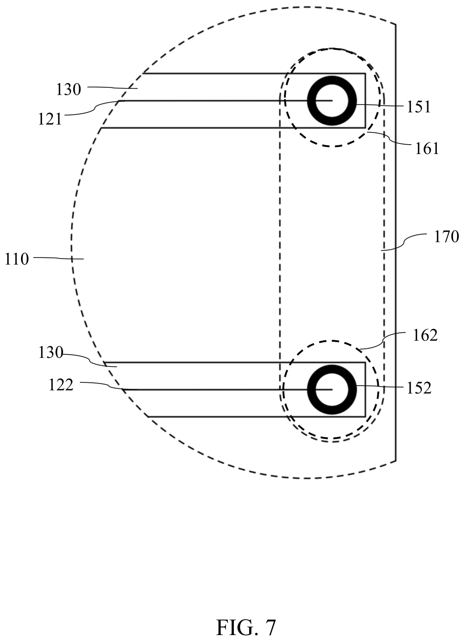

FIG. 7 is a schematic diagram illustrating a structure of a fixing member according to some embodiments of the present disclosure. In some embodiments, as shown in FIG. 7, the base body 110 is fixed with a plurality of metal members. Each of the plurality of metal members corresponds to one electrode 120. For example, the plurality of metal members include a first metal member 151 and a second metal member 152. The first metal member 151 is electrically connected to the first electrode 121, the second metal member 152 is electrically connected to the second electrode 122, and the first metal member 151 and the second metal member 152 are respectively magnetically detachable connected to an external processing circuit, to achieve data transmission between the first electrode 121, the second electrode 122, and the processing circuit. The plurality of metal members (including the first metal member 151 and the second metal member 152) may serve as an interface portion for signal transmission and are configured to transmit signals to the processing circuit. The electrical signals acquired by the plurality of electrodes 120 are transmitted to the processing circuit via corresponding metal members for processing. The processing circuit may be configured for signal processing, storage, transmission, etc. For example, the processing circuit may be configured to process signals acquired by the plurality of electrodes 120. In some embodiments, the plurality of metal members may be implemented as metal buckles, metal rings, metal blocks, or the like, and may be magnetically detachable connected to the processing circuit. When the plurality of metal members are magnetically connected to the processing circuit, the electrical signals acquired by the plurality of electrodes 120 are transmitted to the processing circuit via the plurality of metal members.

In some embodiments, as shown in FIG. 7, one or more fixing members are fixedly disposed on the base body 110. The one or more fixing members are connected to the plurality of metal members and are configured to reinforce the connection between the plurality of metal members and the base body 110. A count of the one or more fixing members may be the same as a count of the plurality of metal members. The one or more fixing members correspond one-to-one with the plurality of metal members. In some embodiments, the one or more fixing members are fixedly laid on a portion of the base body 110 connected to the plurality of metal members. For example, the one or more fixing members may include an embossed transfer film. The embossed transfer film is hot-pressed onto the portion of the base body 110 connected to the plurality of metal members. In some embodiments, an area of the base body 110 covered by the one or more fixing members is greater than an area of the base body 110 covered by the plurality of metal members, thereby avoiding a breakage of the base body 110 during repeated disassembly and assembly of the plurality of metal members and the processing circuit. Exemplarily, the one or more fixing members may include a first fixing member 161 and a second fixing member 162. The first metal member 151 is connected to the first fixing member 161. The second metal member 152 is connected to the second fixing member 162. In some embodiments, a connecting member 170 is provided between the first fixing member 161 and the second fixing member 162. The connecting member 170 connects the first fixing member 161 and the second fixing member 162. The first metal member 151 and the second metal member 152 are simultaneously connected to the processing circuit. In the worn state and during movement, a distance between the first metal member 151 and the second metal member 152 changes due to the elasticity of the base body 110, which may cause a damage to the processing circuit. By disposing the connecting member 170 between the first fixing member 161 and the second fixing member 162, the distance between the first fixing member 161 and the second fixing member 162 is fixed, that is, the distance between the first metal member 151 and the second metal member 152 is fixed, thereby avoiding the damage to the processing circuit. In some embodiments, the first fixing member 161, the second fixing member 162, and the connecting member 170 may be in an integral structure. For example, the first fixing member 161, the second fixing member 162, and the connecting member 170 may all be implemented as the embossed transfer film. Two ends of the embossed transfer film are hot-pressed onto the portion of the base body 110 connected to the metal members to form the first fixing member 161 or the second fixing member 162. A middle portion of the embossed transfer film is further hot-pressed between the two ends (i.e., the first fixing member 161 and the second fixing member 162) of the embossed transfer film. In this case, the first fixing member 161, the second fixing member 162, and the connecting member 170 may be regarded as an integrated unit. Maintaining the structural stability of the integrated unit without deformation can prevent the processing circuit connected to the integrated unit from being damaged.

The basic concepts have been described above. It is apparent to those skilled in the art that the foregoing detailed disclosure is merely exemplary and does not constitute a limitation on the present disclosure. Although not explicitly stated here, those skilled in the art may make various modifications, improvements and amendments to the present disclosure. These alterations, improvements, and modifications are intended to be suggested by the present disclosure, and are within the spirit and scope of the exemplary embodiments of the present disclosure.

Moreover, certain terminology has been used to describe embodiments of the present disclosure. For example, "an embodiment," "one embodiment," and/or "some embodiments" mean a certain feature, structure, or characteristic related to at least one embodiment of the present disclosure. Therefore, it should be emphasized and noted that "an embodiment" or "one embodiment" or "an alternative embodiment" mentioned two or more times in different locations in the present disclosure does not necessarily refer to the same embodiment. In addition, some features, structures, or features in the present disclosure of one or more embodiments may be appropriately combined.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, the present disclosure does not mean that the present disclosure object requires more features than the features mentioned in the claims. Rather, claimed subject matter may lie in less than all features of a single foregoing disclosed embodiment.

In some embodiments, numbers describing quantities of components or attributes are used. It should be understood that such numbers used in the description of the embodiments are modified by the terms "approximately," "approximate," or "substantially" in some examples. Unless otherwise stated, "approximately," "approximate," or "substantially" indicates that the stated number allows a variation of ±20%. Accordingly, in some embodiments, the numerical parameters used in the specification and claims are approximate values. These approximate values can vary depending on the desired characteristics of individual embodiments. In some embodiments, the numerical parameters should consider the specified number of significant digits and apply the method of general digit retention. Although the numerical ranges and parameters used to confirm the breadth of the scope in some embodiments of the present disclosure are approximate values, in specific embodiments, the setting of such numerical values is as precise as possible within a feasible range.

At last, it should be understood that the embodiments described in the present disclosure are merely illustrative of the principles of the embodiments of the present disclosure. Other modifications that may be employed may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A signal acquisition device, comprising a wearable body, wherein the wearable body includes:
a plurality of electrodes in contact with a user's skin, the plurality of electrodes including a first electrode and a second electrode, the first electrode and the second electrode being configured to acquire an electromyography signal (EMG signal) of a target muscle of the user, and the first electrode and the second electrode being arranged at intervals along a muscle fiber direction of the target muscle; and,
a base body, configured to carry the plurality of electrodes and position the plurality of electrodes at the target muscle; wherein,
the base body includes a base yarn, the plurality of electrodes include a conductive yarn, and the base body and the plurality of electrodes are formed by integrated weaving.

2. The signal acquisition device of claim 1, wherein the wearable body further includes one or more waterproof layers, the one or more waterproof layers include a waterproof insulating yarn, the one or more waterproof layers, the base body and the plurality of electrodes are formed by integrated weaving.

3. The signal acquisition device of claim 2, wherein the one or more waterproof layers and the base body are formed by weaving in a coplanar arrangement, and the base body is connected to an outer peripheral side of the one or more waterproof layers.

4. The signal acquisition device of claim 3, wherein the one or more waterproof layers include a plurality of waterproof layers, and each electrode of the plurality of electrodes is connected to an inner peripheral side of a corresponding waterproof layer, respectively.

5. The signal acquisition device of claim 4, wherein the each electrode of the plurality of electrodes and the corresponding waterproof layer are formed by weaving in a coplanar arrangement.

6. The signal acquisition device of claim 4, wherein, in a direction toward the user's skin, each electrode of the plurality of electrodes protrudes beyond the corresponding waterproof layer.

7. The signal acquisition device of claim 3, wherein each electrode of the plurality of electrodes is woven on a side of a corresponding waterproof layer facing the user's skin.

8. The signal acquisition device of claim 2, wherein the one or more waterproof layers are woven on an inner surface of the base body facing the user's skin.

9. The signal acquisition device of claim 8, wherein the plurality of electrodes are woven on a side of the one or more waterproof layers facing away from the base body.

10. The signal acquisition device of claim 8, wherein the one or more waterproof layers include a plurality of waterproof layers, and each electrode of the plurality of electrodes is connected to an inner peripheral side of a corresponding waterproof layer, respectively.

11. The signal acquisition device of claim 3 or 8, wherein concave-convex shapes are woven on surfaces of the one or more waterproof layers; or, concave-convex shapes are woven on surfaces of the plurality of electrodes.

12. The signal acquisition device of claim 1, wherein, in a worn state, the base body includes a proximal end close to a user's heart and a distal end away from the user's heart, and an elasticity of the distal end is greater than an elasticity of the proximal end.

13. The signal acquisition device of claim 12, wherein a thickness of the distal end is greater than a thickness of the proximal end.

14. The signal acquisition device of claim 12, wherein a density of the base yarn at the distal end is greater than a density of the base yarn at the proximal end.

15. The signal acquisition device of claim 1, wherein each electrode of the plurality of electrodes includes a first region and a second region, the first region is connected to the base body through the second region, the first region contacts the user's skin to acquire an electrical signal, and the second region is electrically insulated from the user's skin.

16. The signal acquisition device of claim 15, wherein, in a direction toward the user's skin, the first region protrudes beyond the second region.

17. The signal acquisition device of claim 15, wherein the conductive yarn of the second region is coated with a waterproof insulating film.

18. The signal acquisition device of claim 1, wherein the conductive yarn is at least made by winding a conductive fiber around an elastic fiber; or, the conductive yarn is at least made by coating a periphery of the conductive fiber with an elastic film.

19. The signal acquisition device of claim 1, wherein the base body forms a leg sleeve, the leg sleeve is elastically stretchable along its radial direction, and the plurality of electrodes are configured to acquire the EMG signal of a rear side of a leg of the user.

20. The signal acquisition device of claim 1, wherein the wearable body includes a plurality of straps connected to the base body, the plurality of straps are arranged at intervals along the muscle fiber direction, and, in a worn state, a size of each strap of the plurality of straps is individually adjustable.

21. The signal acquisition device of claim 1, wherein the base body includes an overlapping region overlapping with the plurality of electrodes in a direction perpendicular to the muscle fiber direction, and the base body includes a non-overlapping region staggering the plurality of electrodes in the direction perpendicular to the muscle fiber direction, the overlapping region and the non-overlapping region both extend along the direction perpendicular to the muscle fiber direction, the overlapping region and the non-overlapping region are adjacently distributed along the muscle fiber direction, and an elasticity of the overlapping region is greater than an elasticity of the non-overlapping region.

22. The signal acquisition device of claim 1, wherein a first metal member and a second metal member are fixed to the base body, the first metal member is electrically connected to the first electrode, the second metal member is electrically connected to the second electrode, and the first metal member and the second metal member are respectively magnetically detachably connected to a processing circuit, to achieve data transmission between the first electrode, the second electrode, and the processing circuit.

23. The signal acquisition device of claim 22, wherein a first fixing member and a second fixing member are fixedly provided on the base body, the first metal member is connected to the first fixing member, the second metal member is connected to the second fixing member, and a connecting member is provided between the first fixing member and the second fixing member.
